# EUROPEAN PATENT APPLICATION

(11) **EP 1 797 829 A1**
(43) Date of publication of application: **20.06.2007**
(21) Application number: 06450179.4
(22) Date of filing: 13.12.2006
(51) Int. Cl.: A61B 17/04

(54) **Peek ribbed suture anchor**

(30) Priority: 13.12.2005 US 299666
(71) Applicant: Arthrex, Inc., Naples, Florida 34108 (US)
(72) Inventor: Dreyfuss Peter J, Naples, Florida 34112 (US); Benavitz William C., Naples, Florida 34109 (US); Grafton, Donald, R., Naples, Florida 34112 (US); Schmieding, Reinhold, Naples, Florida 34108 (US)
(74) Representative: Kopecky, Helmut

(57) **Abstract**

A ribbed suture anchor formed of poly-ether ether ketone (PEEK). The suture anchor is radiolucent and revisable. A tapered end disposed on the proximal end of the body can be received into a recess in the distal end of a hand driver. Anchoring ribs are formed along the remaining length of the anchor. The suture is attached to the suture anchor body through an aperture in the anchor.

## Description

### FIELD OF THE INVENTION

The present invention relates to an implant for anchoring surgical suture to bone. More specifically, the present invention relates to a ribbed suture anchor formed of polyether-ether ketone (PEEK).

### BACKGROUND OF THE INVENTION

When soft tissue tears away from bone, reattachment becomes necessary. Various fixation devices, including sutures, screws, staples, wedges, and plugs have been used in the past to secure soft tissue to bone. More recently, various types of threaded suture anchors have been developed.

Suture anchors and implants generally include a structure for attaching or securing the suture to the anchor. U.S. Patent No. 4,632,100, for example, discloses and claims a threaded suture anchor with a complex press-fitted disc and knot structure which secures the suture to the anchor. In other suture anchors, such as those disclosed in U.S. Patent No. 5,370,662, the suture is attached to the anchor by passing the suture through an eyelet at the back end of the anchor. However, the materials used to make such suture anchors can impose limitations on their use. For example, suture anchors made of certain polymers are not visible on magnetic resonance imaging ("MRI") scans. In addition, certain suture anchors may not be revisable once implanted in the bone.

Accordingly, a need exists for a suture anchor or implant formed by a material which is visible on MRI scans and is revisable following implantation. A need also exists for a soft tissue fixation device having a low profile configuration particularly suited for reattachment of tissue to the glenoid rim, for example.

### BRIEF SUMMARY OF THE INVENTION

The suture anchor of the present invention overcomes disadvantages of the prior art, such as those noted above, and achieves the foregoing objectives by providing a suture anchor formed from a material comprising polyether-ether ketone (PEEK).

The PEEK suture anchor of the present invention has a central body, a distal end, and a proximal end. The body preferably has tapered ribs formed along the distal portion, terminating in a blunt or rounded proximal end. The proximal end of the suture anchor body preferably has a round, tapered drive head which is received in a recess of a hand driver. The suture anchor contains an eyelet at the proximal end for receiving a suture strand.

Other features and advantages of the present invention will become apparent from the following description of the invention, which refers to the accompanying drawings.

### BRIEF DESCRIPTION OF THE DRAWINGS

FIG. 1 is a plan view of the suture anchor of the present invention.

FIG. 2 is a sectional elevation of the suture anchor of FIG. 1.

FIG. 3 is a proximal end view of the suture anchor of FIG. 1.

FIG. 4 is a plan view of a hand driver for inserting the suture anchor of the present invention.

FIG. 5 is an elevation view of the hand driver of FIG. 4.

FIG. 6 is a sectional view of the hand driver of FIG. 4.

FIG. 7 is a detail view of the drive end of the hand driver of FIG. 4.

FIG. 8 is a plan view of an alternative hand driver for a method of capsular plication using the suture anchor according to the present invention.

FIG. 9 is an elevational view of the hand driver of FIG. 8.

FIG. 10 is a sectional elevation of the hand driver of FIG. 8.

FIG. 11 is a detail view of the drive end of the hand driver of FIG. 4.

### DETAILED DESCRIPTION OF THE INVENTION

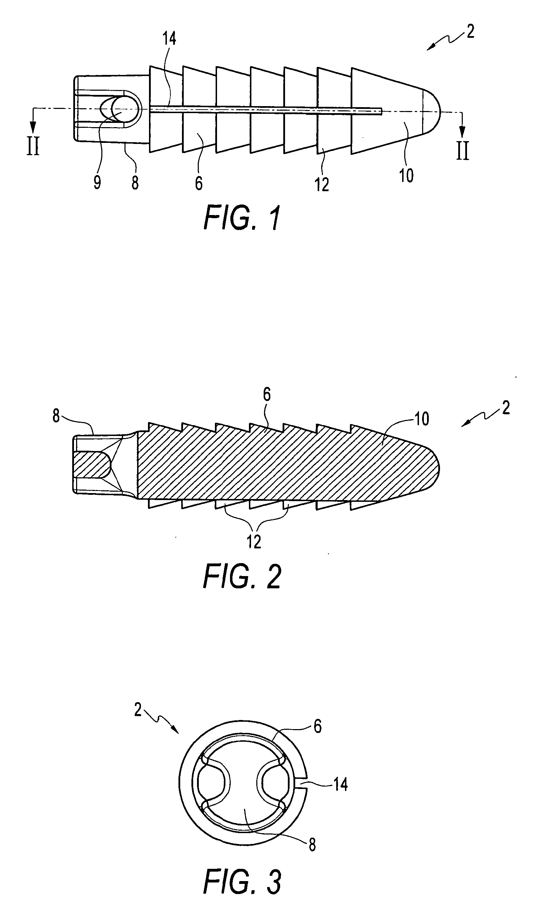

Referring to FIGS. 1-3, an exemplary embodiment of the present invention is shown as a suture anchor 2 having a suture anchor body 6. The suture anchor body 6 preferably is formed of polyether-ether ketone (PEEK).

In a preferred embodiment, anchor body 6 is formed from a material comprising PEEK. A suture anchor formed from a material comprising PEEK has several advantageous properties. First, PEEK is radiolucent and, since it does not contain any metal, it does not produce any metallic scatter on magnetic resonance imaging (MRI) scans.

In addition, suture anchors formed by PEEK have significantly reduced notch sensitivity resulting in a more stable and resilient suture anchor. The construction of an anchor body formed from a material comprising PEEK provides both stable fixation and revisability. Previously available suture anchors may require "wings" or "arms" to provide fixation. In contrast, the ribbed PEEK unibody construction shown in FIGS. 1-3 provide stable fixation without requiring additional structural features. Furthermore, PEEK suture anchors are revisable, for example, by drilling out the anchor. Other advantages of PEEK material are described in a white paper entitled "New Materials in Sports Medicine," Arthrex, Inc., 2005.

The mechanical properties of PEEK closely match the mechanical properties of bone: tensile yield strength, shear strength, and modulus. These properties are not significantly degraded by gamma-irradiation, steam-sterilization (water environment), or oxidation (aging). The material is also resistant to heat and requires no special accommodations for shipping and handling.

Any known type of suture can be selected for use with the suture anchor according to the size of the anchor and the anticipated application. In one embodiment the suture is No. 2 polyester braided suture. In another embodiment, the suture is FiberWire™, a high strength suture formed of a braid of ultrahigh molecular weight polyethylene and polyester, sold by Arthrex, Inc. of Naples, Florida, the assignee of the present application. Advantageously, the lubricity of PEEK allows for easy, non-abrasive sliding of the FiberWire™ suture through the material eyelet.

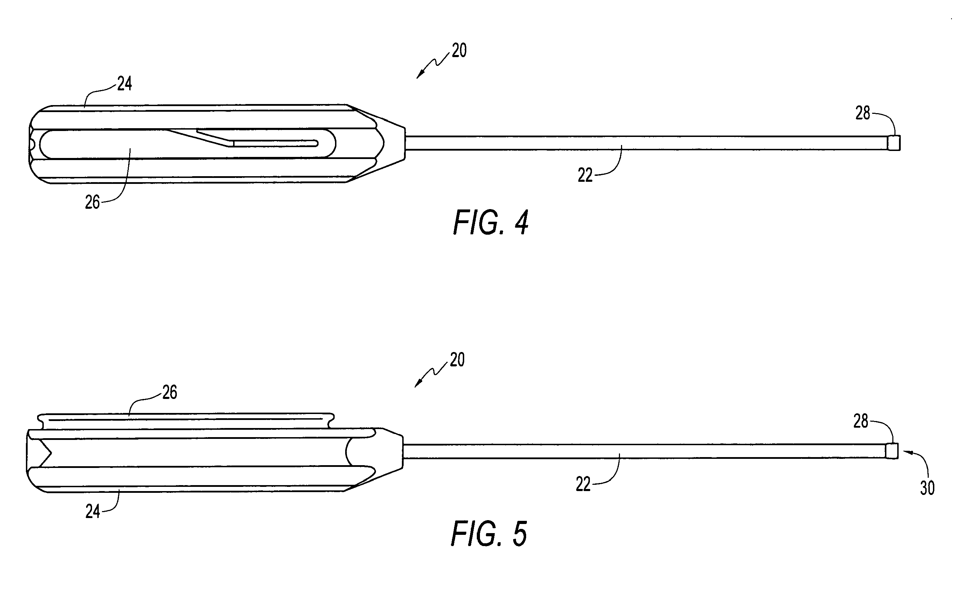

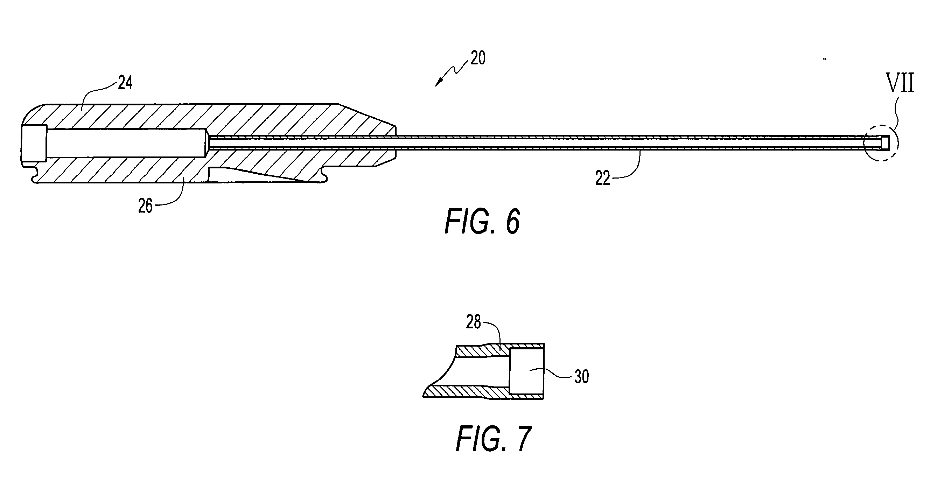

The proximal end 8 of the suture anchor body preferably is tapered for a snug fit into a hand driver described below, for example, with reference to FIGS. 4-7 and includes aperture 9 for receiving the suture in body 6. The distal end 10 of the suture anchor tapers to a blunt tip. Suture anchor 2 is provided with slotted ribs 12 formed circumferentially at least partially around and partially along the length of body 6. Ribs 12 have a truncated, conical shape, each rib increasing in diameter toward the head of the anchor at an angle of preferably 15° with respect to the longitudinal axis of anchor 2, and reaching a major diameter of 3.0 mm. Slots 14 are formed in ribs 12 on alternating sides of body 6.

Referring to FIGS. 4-7, a hand driver 20 according to the present invention is shown. Hand driver 20 includes a cannulated shaft 22 with a cannulated handle 24. A cleat 26 is provided on the handle for securing suture attached to the suture loop on the suture anchor and passed through the cannulated shaft and handle. The distal tip 28 of cannulated shaft 22 provides a recess 30 which receives the proximal end of suture anchor 2. The outer diameter of the distal end of the driver preferably is less than or equal to the maximum outer diameter of the suture anchor.

The suture anchor is urged into a hole formed in bone. The hole can be formed by punching or boring, for example. The ribs secure the anchor in the bone. The slots enhance attachment in the bone and support bony in-growth for increased pull out strength.

Advantageously, the hole formed in bone is made deep enough, and the suture anchor is advanced into the hole sufficiently, so that the proximal end of the anchor sits flush with or below the bone surface. Accordingly, the repair leaves a smooth bone surface, minimizing or eliminating abrasion or other damage to surrounding soft tissue. The anchor generally becomes encapsulated by fibrous tissue within six weeks after implantation.

Although PEEK is the most preferred material for the suture anchor of the present invention, other radiolucent and revisable materials known in the art can be utilized. The suture anchor of the present invention can be molded or formed by standard machining techniques to provide the features and structures of the suture anchor described herein.

The suture anchor of the present invention is particularly well suited for reattachment of the glenoid labrum or inferior glenohumeral ligament in patients with primary or recurrent anterior dislocation or subluxation of the shoulder in association with adequate post-operative immobilization. More specifically, the anchor also can be used for repair procedures such as capsulabral plication, as described below.

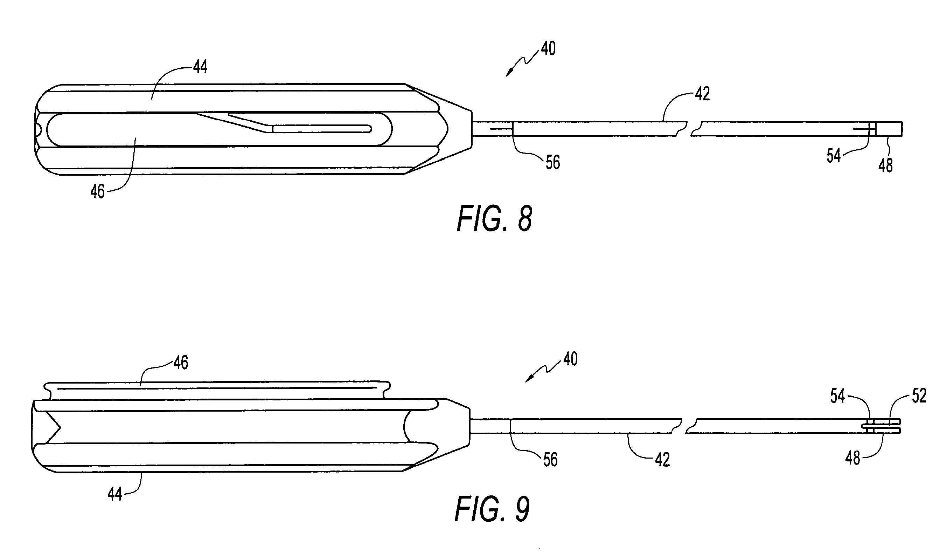

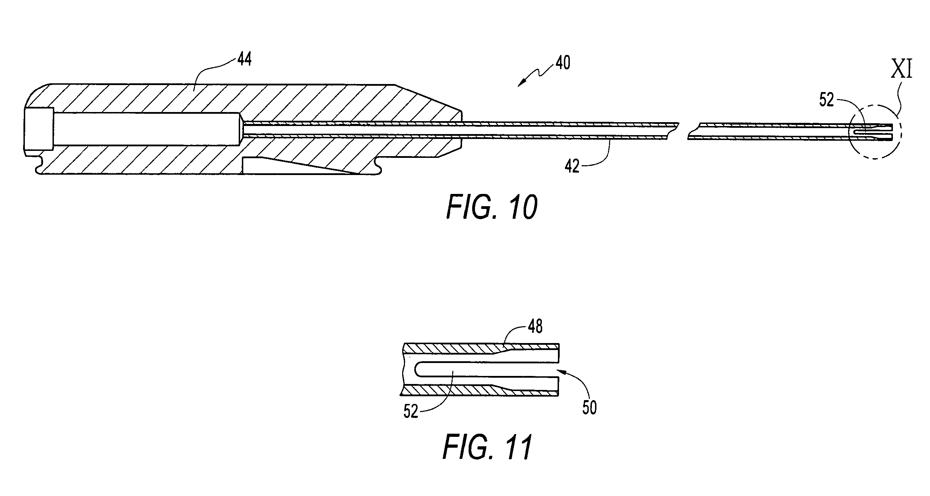

Referring to FIGS. 8-11, a driver 40 for capsule plication using the anchor according to the present invention is shown. Capsulolabral plication is indicated for repair of certain types of shoulder laxity. When pathologically increased anterior laxity is combined with a Bankart lesion, for example, the addition of a capsular plication to the reattachment of the capsulolabral avulsion has been recommended.

Driver 40 includes a cannulated shaft 42 with a cannulated handle 44. A cleat 46 is provided on the handle for securing suture attached to the suture loop on the suture anchor and passed through the cannulated shaft and handle. The distal tip 48 of cannulated shaft 42 provides a recess 50 which receives the proximal end of suture anchor 2. The outer diameter of the distal end of the driver preferably is less than or equal to the maximum outer diameter of the suture anchor. Driver 40 also features a slot 42 which is continuous with recess 50.

The method of capsular plication proceeds using a 36-inch (91.4 cm) long #2 suture to plicate the capsulolabral complex. Both free ends of the suture are brought out an operative cannula. A spear with an included obturator is introduced through a skin incision or a clear cannula. The tip of the spear is positioned on bone and the obturator is removed.

A pilot hole is prepared in bone using either a punch or a drill depending on surgeon preference. With the manual punch, a mallet is used to advance the punch into bone until the punch handle meets the back of the spear and/or the shoulder on the distal part of the punch meets the bone surface. Alternatively, the drill can be attached with a Jacob chuck to a motorized drill and advanced until the stop on the drill bit meets the back of the spear.

After the pilot hole is created and the punch or drill is removed, the sterile-packaged implant 2 is opened to the sterile field using appropriate sterile technique. The implant is removed from the standard hand driver 20 and the suture is unloaded from the implant. A separate sterile packaged plication driver 40 is opened to the sterile field. One of the two legs of the plication suture is selected. This suture leg is the one on the medial side, or the one that passes under the tissue.

The selected suture leg is loaded through the implant eyelet. The implant 2 is positioned on plication driver 40 so that the open side of the eyelet 4 faces the open slot 52 on the driver. The suture leg will exit the slot 52 on the driver 40. The implant with driver is introduced into the prepared pilot hole by hand. A mallet then is used to advance the implant into the hole. The implant is advanced until a second laser line 54 on the distal tip of the driver is flush with the bone surface and a laser line 56 on the proximal part of the implant driver shaft is flush with the back of the spear handle.

The implant driver handle is pulled straight off the implant and the spear is removed. Additional implants are inserted dependent upon the size of the soft tissue defect. Suture passing and knot tying are carried out in the preferred fashion.

Although the present invention has been described in relation to particular embodiments thereof, many other variations and modifications and other uses will become apparent to those skilled in the art.

## Claims

1. A ribbed suture anchor comprising:
an anchor body formed of a material comprising polyether-ether ketone polymer; and
plurality of ribs formed circumferentially at least partially around and at least partially along the length of the suture anchor body.

2. The ribbed suture anchor of claim 1, wherein the ribbed suture anchor body has at least one aperture for receiving a suture strand.

3. The ribbed suture anchor of claim 2, further comprising a suture strand, wherein the suture strand is inserted through the aperture of the ribbed suture anchor body.

4. The ribbed suture anchor of claim 1, wherein the suture anchor is radiolucent.

5. The ribbed suture anchor of claim 1, wherein the distal end of the suture anchor body tapers to a blunt tip.

6. The ribbed suture anchor of claim 1, wherein the ribs have a truncated, conical shape.

7. The ribbed suture anchor of claim 1, where the ribs further comprise slots formed in the ribs.

8. A ribbed suture anchor assembly comprising:
a hand driver having a cannulated shaft with an open recess on an end of the shaft; and
a ribbed suture anchor formed of a material comprising polyether-ether ketone positioned in the recess on the end of the shaft.
